(19) Europäisches Patentamt
European Patent Office
Office européen des brevets

(11) Publication number: 0 128 745
B1

(12) **EUROPEAN PATENT SPECIFICATION**

(45) Date of publication of patent specification: 24.09.86

(51) Int. Cl.[4]: C 07 C 47/07

(21) Application number: 84303844.9

(22) Date of filing: 06.06.84

# (54) Process for producing acetaldehyde.

(30) Priority: 13.06.83 JP 104291/83
05.03.84 JP 41800/84

(43) Date of publication of application:
19.12.84 Bulletin 84/51

(45) Publication of the grant of the patent:
24.09.86 Bulletin 86/39

(84) Designated Contracting States:
BE CH DE FR GB IT LI

(56) References cited:
EP-A-0 106 590
DE-B-1 197 071
US-A-4 321 211

(73) Proprietor: BABCOCK-HITACHI KABUSHIKI KAISHA
6-2, 2-chome, Ohtemachi Chiyoda-ku
Tokyo (JP)

(72) Inventor: Nishimura, Yasuyuki Kure Research Laboratory
Babcok-Hitachi K.K. 3-36, Takara-machi
Kure-shi Hiroshima-ken (JP)
Inventor: Yamada, Mutsuo Kure Research Laboratory
Babcok-Hitachi K.K. 3-36,Takara-machi
Kure-shi Hiroshima-ken (JP)
Inventor: Arikawa, Yoshijiro Kure Research Laboratory
Babcok-Hitachi K.K. 3-36, Takara-machi
Kure-shi Hiroshima-ken (JP)
Inventor: Kamiguchi, Taiji Kure Research Laboratory
Babcok-Hitachi K.K. 3-36, Takara-machi
Kure-shi Hiroshima-ken (JP)
Inventor: Kuwahara, Takanori Kure Research Laboratory
Babcok-Hitachi K.K. 3-36, Takara-machi
Kure-shi Hiroshima-ken (JP)
Inventor: Tanimoto, Hirotoshi Kure Research Laboratory
Babcok-Hitachi K.K. 3-36, Takara-machi
Kure-shi Hiroshima-ken (JP)

EP 0 128 745 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

0 128 745

(74) Representative: **Pendlebury, Anthony et al**
**Page, White & Farrer 5 Plough Place**
**New Fetter Lane**
**London EC4A 1HY (GB)**

**Description**

This invention relates to a process for producing acetaldehyde, and more particularly it relates to a process for producing acetaldehyde by oxidising ethylene with oxygen in the presence of a metal complex catalyst.

Acetic acid and aldehydes as basic chemicals for the petrochemical industry have been produced by oxidation reactions of the corresponding raw materials. Such oxidation reactions occupy an important place among reaction processes which are employed in the petrochemical industry. These oxidation reactions have so far been carried out at high temperatures and high pressures, but improvement of reaction selectivity and yield is becoming an important problem to be settled.

Acetaldehyde is an important, basic material for production of many organic substances, and its derivatives include many compounds such as acetic acid, acetic esters, etc. Acetaldehyde is normally produced by acetylene hydration processes, ethanol dehydrogenation processes and ethylene direct oxidation processes. However, acetylene hydration processes and ethanol dehydrogenation processes are not employed as commercial processes since byproducts are formed in a large amount due to the severe conditions. In contrast to these processes, a so-called Wacker's process wherein ethylene is used as raw material and palladium chloride ($Pd(II)Cl_2$)-cupric chloride ($Cu(II)Cl_2$) is used as catalyst, has been noted as a process for producing acetaldehyde under mild reaction conditions, and has come to be the main process among current acetaldehyde production processes.

According to the process, a composite catalyst obtained by dissolving $Pd(II)Cl_2$ and $Cu(II)Cl_2$ as catalysts in a hydrochloric acid solution (pH:0—2) is employed. Ethylene is first oxidised with divalent palladium Pd(II) and water ($H_2O$) to form acetaldehyde ($CH_3CHO$). The reaction is expressed by the following equation wherein water participates in the reaction:

$$CH_2 = CH_2 + Pd(II)Cl_2 + H_2O \rightarrow CH_3CHO = Pd(O) \rightarrow + 2HCl \quad (1)$$

As seen from the above reaction equation, Pd(II) is reduced to metal palladium Pd(O) which precipitates. This is prevented by making $Cu(II)Cl_2$ coexistent in a large amount, and at the same time, Pd(O) is oxidised into Pd(II) for regeneration according to the following equation:

$$Pd(O) + 2Cu(II)Cl_2 \rightarrow Pd(II)Cl_2 + 2Cu(I)Cl \quad (2)$$

Further, slightly soluble Cu(I)Cl produced as a byproduct is oxidised with oxygen in the presence of HCl and $Cu(II)Cl_2$ is regenerated according to the following equation:

$$2Cu(I)Cl + 1/2O_2 + 2HCl \longrightarrow 2CuCl_2 + H_2O \quad (3)$$

As described above, by employing a redox system of Pd(II)/Pd(O) and Cu(II)/Cu(I), continuous oxidation of ethylene is rendered possible. However, according to this process, molecular oxygen is not directly reacted with ethylene as described above, but since complicated oxidation-reduction reactions of Pd(II)/Pd(O)—Cu(II)/Cu(I) system are involved, these reactions constitute a reaction rate-determining step. Further, since slightly soluble Pd(O) and Cu(I)Cl are formed midway during the reactions, a concentrated HCl aqueous solution having a high concentration (pH0—2) must be used; hence it is necessary to select corrosion-resistant materials in which to perform the process. Further, since oxygen has a low solubility in water, in order to accelerate the reaction by increasing the amount of dissolved oxygen, it is necessary to carry out the reaction under pressure and heating conditions such as 10 $Kg/cm^2$ and 100°C. Furthermore, when dissolved oxygen in excess is released into the gas phase, ethylene mixes with oxygen, which is hazardous and can cause an explosion; hence safety measures must be taken.

The object of the present invention is to provide a process for producing acetaldehyde by oxidising ethylene with oxygen at lower temperatures and lower pressures, selectively and with a high yield.

The present invention, in short, resides in a process wherein using a composite catalyst comprising a complex of a transition metal capable of forming an oxygen complex by coordination of oxygen molecule with the metal ion of the transition metal, and a complex of a transition metal capable of forming an ethylene complex by coordination of ethylene with the metal ion of the transition metal, ethylene activated by forming the ethylene complex is oxidised with the combined oxygen in the oxygen complex, activated by forming the oxygen complex, to produce acetaldehyde under mild conditions, selectively and with a high yield.

Accordingly, the present invention provides a process for producing acetaldehyde by oxidising ethylene with oxygen in the presence of a metal complex catalyst, characterised in that the metal complex catalyst is a composite catalyst comprising a first metal complex catalyst of formula $M_mX_n.L_l$ capable of forming an oxygen complex by coordination thereof with oxygen,

wherein M represents a transition metal belonging to at least one group selected from Groups I and IV to VII and from the iron group in Group VIII of the Periodic Table; X represents an anion; ligand L represents an organic phosphorous compound; and each of m, n and l independently represents a positive integer from 1 to 4,

and a second metal catalyst of formula $M'_{m'}\ X_{n'}.L'_{l'}$ capable of forming an ethylene complex by coordination thereof with ethylene,

wherein M' represents a transition metal of the platinum group in Group VIII of the Periodic Table; X represents an anion; ligand L' represents at least one compound selected from a nitrile, an organic fluorine compound, and alkoxy, alkyl and amide derivatives of phosphorus acid and phosphoric acid; and each of m', n', and l' independently represents a positive integer from 1 to 4,

the process comprising contacting the first metal complex catalyst with oxygen to form an oxygen complex, contacting the second metal complex catalyst with ethylene to form an ethylene complex, and reacting together the said oxygen complex and the said ethylene complex to produce acetaldehyde.

Various studies have been made on Cu(I)-protein and Fe(II)-protein complexes in living bodies, as typical examples of oxygen complexes capable of functioning as effective oxidising agents for oxidation reaction of various kinds of organic substances. However, there are very few examples of oxygen complexes which are capable of being utilised on a commercial scale. We have made extensive research on stable oxygen complexes applicable to oxidation of organic substrates with safety. As a result, we have found that, as a representative example, a solution of a complex (Cu(I)Cl·hmpa) resulting from the reaction of cuprous chloride (Cu(I)Cl) with hexamethyl-phosphoramide (hereinafter abbreviated to hmpa; also known as tris(dimethylamino) phosphine oxide) (Japanese Patent Application Laid-Open Nos. 56—118720 and 57—19017, as disclosed as an absorbing solution for carbon monoxide (CO) reacts with oxygen in a molar ratio of 2:1 in contact therewith to form the following oxygen complex:

$$2Cu(I)Cl\cdot hmpa + O_2 \rightarrow (Cu(I)Cl\cdot hmpa)_2.O_2 \qquad (4)$$

The oxygen coordinated with the transition metal is activated and is the same as in the case of an oxygen complex of Cu(I)-protein or Fe(II)-protein. A complex such as Cu(I)Cl·hmpa can be expressed by the general formula $M_mX_n\cdot L_l$ in which m = 1, n = 1 and l = 1. Further, for example, when the metal atom M is Ti(III) or V(III) and the anion is $C1^-$, the resulting complex is $Ti(III)Cl_3\cdot hmpa$ or $V(III)Cl_3\cdot hmpa$, with m = 1, n = 3 and l = 1 in the above-mentioned general formula.

The above new complexes are so stable that boiling at 100°C is required for oxidising Cu(I) into Cu(II) by the combined oxygen. Further, the oxygen once absorbed in the form of the oxygen complex is not easily separated even by heating or deaeration under reduced pressure; hence the absorption is irreversible. Due to this specific feature of the complex, after it has been contacted with pure oxygen or air to form an oxygen complex, it becomes possible to remove physically dissolved free oxygen from the oxygen complex solution by heating or deaeration in vacuo; hence this results in a great effectiveness in the aspect of safety. Another specific feature of the complex is that oxygen is selectively absorbed from air to form all the same oxygen complex as in the case of pure oxygen.

According to the present invention, ethylene is oxidised with the combined oxygen activated by forming an oxygen complex, as described above, and if ethylene is also activated by formation of an ethylene complex, the process can be performed at lower temperatures and pressures than normal. We have made studies on various complexes of transition metals belonging to platinum group. In the case of palladium chloride $Pd(II)Cl_2$ as a representative example, this compound forms a complex having two molecules of hmpa coordinated therewith, as shown in the following equation, and dissolves well therein:

$$Pd(II)Cl_2 + 2hmpa \rightleftarrows Pd(II)Cl_2\cdot(hmpa)_2 \qquad (5)$$

This complex can be expressed by the general formula $M'_{m'}X_{n'}\cdot L'_{l'}$, wherein m' = 1, n' = 2 and l' = 2.

When ethylene is passed through the complex, an ethylene complex is formed, which reaction is shown in the following equation:

$$Pd(II)Cl_2\cdot(hmpa)_2 + CH_2 = CH_2 \rightleftarrows Pd(II)Cl_2\cdot C_2H_4\cdot hmpa + hmpa \qquad (6)$$

However, since the coordination of this ethylene complex is weak, various studies have been made on a complex capable of forming a more stable ethylene complex.

As a result, in a representive example, when a nitrile such as acetonitrile is added as a modifying ligand (an auxiliary complexing agent), to a hmpa complex solution of $Pd(II)Cl_2$, the following new complex is formed:

$$Pd(II)Cl_2\cdot(hmpa)_2 + CH_3CN \rightleftarrows Pd(II)Cl_2\cdot CH_3CN\cdot hmpa + hmpa \qquad (7)$$

When ethylene is passed through this complex, a stable ethylene complex is formed as shown by the following equation:

$$Pd(II)Cl_2\cdot CH_3CN\cdot hmpa + CH_2 \rightleftarrows = CH_2Pd(II)Cl_2\cdot C_2H_4\cdot CH_3CN + hmpa \qquad (8)$$

With such a stable ethylene complex, ethylene is notably activated.

Such a formation of a complex of the above new Pd(II) complex with ethylene has been studied by a

gas absorption method. It was found that an hmpa solution of acetonitrile absorbed 0.07 mol/l of ethylene at 20°C under an ethylene partial pressure of 1 atm, whereas the solution of $Pd(II)Cl_2 \cdot CH_3CN \cdot hmpa$ complex absorbed 0.10 mol/l of ethylene which is about 1.5 times the above amount. Although ethylene is absorbed in a large amount even only due to the solvent used therein since the system is non-aqueous, the above difference in the amount of ethylene absorbed evidences that a new complex of a Pd(II) complex with ethylene has been formed.

Thus in a typical embodiment of the present invention ethylene coordinated with a Pd(II) complex in the form of an ethylene complex and activated thereby is oxidised with the combined oxygen contained in an oxygen complex as described above, under mild conditions to produce acetaldehyde.

According to the present invention, typically, a two-component system catalyst consisting of $Cu(I)Cl \cdot hmpa$ complex and $Pd(II)Cl_2 \cdot CH_3CN \cdot hmpa$ complex is dissolved in liquid hmpa (also as a ligand) or toluene etc., each as a solvent, followed by passing air through the resulting solution so as to give an adequate oxygen complex concentration, as decribed below, to form an oxygen complex, removing excess oxygen by heating, deaeration or the like, passing ethylene through the resulting complex, to form an ethylene complex, and oxidising the thus activated ethylene with the combined oxygen contained in the oxygen complex, at a temperature close to room temperature to produce acetaldehyde nearly quantitatively.

This oxidation reaction is expressed by the following equation when hmpa is used as a complexing agent and as a solvent:

$$(Cu(I)Cl \cdot hmpa)_2O_2 + 2Pd(II)Cl_2 \cdot C_2H_4 \cdot CH_3CN + hmpa \rightarrow 2CH_3CHO + 2Cu(I)Cl \cdot hmpa + 2Pd\,(II)Cl_2 \cdot CH_3CN \cdot hmpa \quad (9)$$

As described above, ethylene coordinated with Pd(II) complex is oxidised with molecular oxygen coordinated with the Cu(I) complex. Thus, the valences of the metal ions in the complexes are unchanged, and water ($H_2O$) does not participate in the acetaldehyde-producing reactions. In the present invention, however, it does not matter if water is present, so long as its amount is such that no precipitate is formed. Even in view of this fact, the present process is quite different from Wacker's process wherein an oxidation-reduction reaction by way of Pd(II) ion and water takes place. As for the complex of the present invention, where air is passed therethrough after completion of the reaction, the oxygen complex is regenerated to make it possible to reuse the complex as the catalyst for ethylene oxidation. Further, the Pd(II) complex, too, may be repeatedly used as the catalyst for ethylene activation.

Thus, according to the present invention, since the reaction substrate is activated in the form of a complex, it is possible to attain a reaction rate superior to that attained with conventional processes, under normal pressures and at a low temperature close to room temperature.

Preferred compounds of formula $M_mX_n$ for forming the complex catalyst $(M_mX_n \cdot L_l)$ which is capable of forming the oxygen complex in the composite catalyst system are salts of Cu or Ag of Group I, Ti or Zr of Group IV, V or Nb of Group V, Cr, Mo, or W of Group VI, Mn of Group VII and Fe, Co or Ni of Group VIII, of the Periodic Table; halides of Cu(I), Ti(III) or V(III) are particularly preferred. Preferred anions X in the complex catalyst are $Cl^-$, $Br^-$, $I^-$, $BF_4^-$, $PF_4^-$, $PF_6^-$, $SO_4^{2-}$ and $CH_3COO^-$. The ligand L is preferably an organic phosphorus compound represented by phosphoric acid derivatives such as triphenylphosphine oxide, hexamethylphosphoramide and mono-, di- and triesters formed by reaction of phosphoric acid with methanol, ethanol or the like; dimethyl methylphosphinate, methyl dimethylphosphinate; and phosphorus acid derivatives such as mono-, di- or triesters formed by reaction of phosphorus acid with methanol, ethanol, or the like, phenyl phosphonous acid esters, dimethylphosphinic acid esters, triethylphosphine, triphenylphosphine. Hexamethylphosphoramide (hmpa) is particularly preferred.

Preferred compounds of formula $M'm'X_n'$ for forming the complex catalyst $(M'_{m'}\ X_{n'} \cdot L'_{l'})$ capable of forming the ethylene complex are salts of lower valence ions of transition metals belonging to platinum group in Group VIII; particularly preferred are halides of Pd(II) or Pt(II). The ligand L' is preferably a nitrile such as acetonitrile, propionitrile, benzonitrile, tolunitrile, etc., or the above-mentioned organic phosphorus compounds or organic fluorine compounds such as fluorinated toluene, benzotrifluoride, etc.; the nitriles are particularly preferred.

Preferred solvents for use when the reaction is carried out in solution are those which dissolve the composite complex and at the same time are easily separated from the resulting acetaldehyde (b.p. 21°C/760mmHg). Examples of typical solvents are n-hexane, toleune, cyclohexane, methyl isobutyl ketone, cyclohexanone, ethanol, ethylene glycol, butyl acetate, propylenecarbonate, chloroform, chlorobenzene, pyridine, tetrahydrofuran, etc., or mixtures thereof. The compound used as the ligand L and L' may also be used as a solvent if the said compound is a liquid.

The oxidation process may proceed further to form acetic acid, depending on the reaction conditions. In such a case, the product is a mixture of acetaldehyde and acetic acid, and acetaldehyde may be separated easily from acetic acid through distillation.

In the present invention, in order to improve the selectivity of acetaldehyde production, it is preferred to include a basic compound in the reaction system, as described later in Examples. Examples of the suitable such basic compounds are sulfolane, dimethylsulfolane, dimethylsulfoxide, dimethylformamide, trimethylmethane, dimethylsulfone, etc.

The present invention will be illustrated further by the following Examples. The gas volumes quoted in the Examples are obtained under standard conditions.

Example 1

Into a 1l capacity reactor were fed cuprous chloride (hereinafter referred to as Cu(I)Cl) (5g, 50mmols) and hmpa (325g) to prepare Cu(I)Cl·hmpa complex solution (330ml). Separately, into a test tube with a ground stopper were fed palladium chloride (Pd(II)$Cl_2$) (1.3g, 7mmols) and acetonitrile ($CH_3CN$) (130g) to prepare Pd(II)$Cl_2$·$(CH_3CN)_2$ complex solution (170ml). This solution was transferred into the above reactor to prepare a catalyst solution (Cu(I)Cl·hmpa/Pd(II)—$Cl_2$·$CH_3CN$·hmpa/hmpa, $CH_3CN$ system) (500ml) containing Cu(I)Cl (0.1mol/l) and Pd(II)$Cl_2$ (0.015mol/l). When air (800ml) was passed through this solution at 25°C under atmospheric pressure, oxygen (147ml, 6mmols) was absorbed to obtain a solution of an oxygen complex having as concentration of 0.01mol/l. Thereafter, when nitrogen gas was passed therethrough, only the oxygen physically dissolved in the liquid phase of the reactor was removed, but separation of oxygen from the combined oxygen was not observed. Namely, the oxygen absorption is irreversible. This is very advantageous with respect to safety in practical processes.

After this operation, ethylene (2,200ml) was passed therethrough similarly at 25°C under atmospheric pressure, and ethylene (1,960ml, 80mmols) was absorbed to give an ethylene concentration in the solution of 0.16mol/l.

Immediately thereafter the solution was warmed to 60°C and subjected to reaction for 30 minutes, followed by cooling the reaction solution. After analysing the resulting product according to gas chromatography, $CH_3CHO$ (0.4g, 9.6mmols) was produced.

The reaction of the ethylene complex with the oxygen complex is carried out according to the above equation (9), and in this Example, the ethylene complex is present in excess relative to the oxygen complex. Thus, the conversion of ethylene into acetaldehyde in this Example was 86% based on the combined oxygen in the oxygen complex.

Example 2

Example 1 was repeated except that acetonitrile was replaced by benzonitrile. The yield of acetaldehyde was 98%, that is, a higher yield than that obtained when using acetonitrile.

Example 3

The reaction in Example 2 was carried out for 2 hours. The yield of $CH_3CHO$ fell to 93%, and acetic acid (5%) was formed.

Example 4

Example 2 was repeated except that hmpa (105g), benzonitrile (15g) and sulfolane (480g) were added and reaction was carried out for one hour. The yield of $CH_3CHO$ amounted to 98%, and even after additional 5 hours, oxidation into acetic acid was not observed.

Example 5

Reaction was carried out under the same conditions as in Example 4 except that Pd(II)$Cl_2$ in Example 1 was replaced by Pt(II)$Cl_2$. The yield of acetaldehyde was 99%; therefore, the oxidation reaction proceeded nearly quantitatively.

Example 6

Example 2 was repeated except that 85g of hmpa was used and toluene (275g) was added, to study the effect of solvent. The yield of acetaldehyde was 97%; i.e., almost the same as in Example 2.

Example 7

Example 2 was repeated except that Cu(I)Cl was replaced by cuprous bromide (Cu(I)Br). The yield of acetaldehyde was 96%.

Example 8

Example 2 was repeated except that Cu(I)Cl was replaced by cuprous iodide (Cu(I)I). The yield of acetaldehyde was 97%.

Example 9

Example 4 was repeated except that Cu(I)Cl was replaced by V(III)$Cl_3$. The yield of $CH_3CHO$ was 60%. Further, in the case of replacement by Ti(III)$Cl_3$, the yield of $CH_3CHO$ was 72%.

Example 10

Example 4 was repeated except that benzonitrile was replaced by benzotrifluoride. The yield of $CH_3CHO$ was 89%.

Example 11

Beads of a styrene-divinylbenzene copolymer of macroreticular form (Amberlite (Trademark) XA D—4 made by Organo Company, particle diameter 1mmφ; specific surface area 700—800 $m^2/g$) (50ml) was impregnated with a catalyst solution containing the oxygen complex having the composition shown in Example 4, followed by filtration by means of suction to prepare a granular catalyst, which was then filled in a hard glass reaction tube having an inner diameter of 20 mmφ. The tube was heated to 60°C, ethylene (1l/min, SV = 1,200 $h^{-1}$ was passed through and the acetaldehyde contained in the exit gas was analysed. The resulting product was acetic acid alone, and its yield based on ethylene was 4% until two hours since the start of the reaction. Thereafter the exit gas was recycled to obtain an acetaldehyde yield of 87% based on the combined oxygen in the oxygen complex. Further, the feed of ethylene was once stopped, followed by passing air therethrough to regenerate the combined oxygen consumed by the reaction and then again carrying out oxidation experiment under the above conditions to obtain similar results.

From the foregoing, it is evident that even when the complex catalyst of the present invention is supported on a carrier, the reaction by means of the combined oxygen in the oxygen complex proceeds.

In addition, it was possible to use other porous carriers such as silicates, active carbon, porous glass, etc. Further, as for the treating process after the impregnation of the catalyst solution, various processes other than filtration by means of suction may be employed such as passing of heated gas, low temperature calcination, etc.

Comparative Example 1

In Examples 1, 2, 3, 4 and 10, similar catalyst solutions were prepared except that neither nitrile nor organic fluorine compound was added, followed by carrying out the same operations. The yields of $CH_3CHO$ were 0.1% or less. Thus it was found that nitriles or organic fluorine compounds as a modifying ligand contributed greatly to ethylene activation.

Comparative Example 2

Into a reactor as described in Example 1 were fed $Pd(II)Cl_2$ (1.3g) and hmpa (325g) to prepare a hmpa solution of $Pd(II)Cl_2 \cdot ^{-}(hmpa)_2$. Ethylene was passed through the solution in the same manner as in Example 1 but without passing air therethrough, to carry out reaction under the same conditions as in Example 1 (60°C, 30 minutes). The ethylene was not oxidised. Further, no precipitate of metal palladium (Pd(O)) was formed; this shows that oxidation by means of Pd(II) ion did not occur.

Comparative Example 3

Cu(I)Cl (5g) was added to the complex solution prepared in Comparative Example 2, to prepare a catalyst solution consisting of $Cu(I)Cl/Pd(2)Cl_2$/hmpa, followed by carrying out the same procedure and reaction as in Comparative Example 2. No ethylene oxidation was observed. This shows that it is necessary to pass air through the solution thereby to form an oxygen complex.

Comparative Example 4

Benzonitrile was added to the complex solution prepared in Comparative Example 3, followed by carrying out the same procedure and reaction as in Comparative Example 1. In this case, too, since air was not passed, no ethylene oxidation was observed.

Comparative Example 5

In comparative Example 2, air was passed, but ethylene did not react. This shows that oxidation by means of free oxygen does not occur in the present system.

Comparative Examples 2 and 3, show that the present invention is entirely different from the process for producing acetaldehyde from ethylene by the use of Pd(II)Cl—Cu(II)Cl redox system as catalyst.

In addition, when air was passed through the catalyst solution containing the ethylene complex in Comparative Example 4, acetaldehyde was produced with a high yield as in the above Examples.

From the foregoing, it is evident that unlike conventional processes, the present invention is a new process for producing acetaldehyde by oxidising the combined ethylene activated by formation of an ethylene complex, with the combined oxygen activated by formation of an oxygen complex.

According to the present invention, ethylene is not directly contacted with oxygen gas, but ethylene and oxygen each coordinated with a transition metal ion and activated thereby through a specified composite catalyst system are reacted together; hence the reaction can be carried out at low temperatures and low pressures such as nearly room temperature and atmospheric pressure thereby to produce the desired acetaldehyde selectively and in high yield. Further, according to the present invention, since the amount of byproducts is small, it is possible to simplify the production steps including the subsequent purification step. Further, even when air is used as oxygen source, oxygen is selectively absorbed; hence the same effectiveness as in the case of use of pure oxygen gas is obtained. Furthermore, since the oxygen absorption is irreversible, it is possible to easily remove excess free oxygen after the oxygen complex has been formed; hence this process is very advantageous also in the aspect of safety.

**Claims**

1. A process for producing acetaldehyde by oxidising ethylene with oxygen in the presence of a metal complex catalyst, characterised in that the metal complex catalyst is a composite catalyst comprising a first metal complex catalyst of formula $M_mX_n \cdot L_l$ capable of forming an oxygen complex by coordination thereof with oxygen.

wherein M represents a transition metal belonging to at least one group selected from Groups I and IV to VII and from the iron group in Group VIII of the Periodic Table; X represents an anion; ligand L represents an organic phosphorous compound; and each of m, n, and l independently represents a positive integer from 1 to 4,

and a second metal complex catalyst of formula $M'_{m'} X_{n'} \cdot L'_{l'}$ capable of forming an ethylene complex by coordination thereof with ethylene,

wherein M' represents a transition metal of the platinum group in Group VIII of the Periodic Table; X represents an anion; ligand L' represents at least one compound selected from a nitrile, an organic fluorine compound, and alkoxy, alkyl and amide derivatives of phosphorus acid and phosphoric acid; and each of m', n', and l' independently represents a positive integer from 1 to 4,

the process comprising contacting the first metal complex catalyst with oxygen to form an oxygen complex, contacting the second metal complex catalyst with ethylene to form an ethylene complex, and reacting together the said oxygen complex and the said ethylene complex to produce acetaldehyde.

2. A process according to Claim 1, wherein X is an anion selected from $Cl^-$, $Br^-$, $I^-$, $BF_4^-$, $PF_6^-$, $SO_4^{2-}$ and $CH_3COO^-$ anions.

3. A process according to Claim 1 or 2, wherein ligand L is at least one compound selected from alkoxy, alkyl and amide derivatives of phosphorus acid and phosphoric acid.

4. A process according to any one of the preceding Claims, wherein as a solvent for said first metal complex and for said second metal complex, there is at least one compound selected from aliphatic, aromatic and alicyclic hydrocarbons, oxygen-containing organic compounds, organic halide compounds, and nitrogen-containing organic compounds.

5. A process according to any one of the preceding Claims, wherein the ligand L or L' is a liquid and the compound used as the ligand L or L' is also used as a solvent for said complexes.

6. A process according to any one of the preceding Claims, wherein oxygen or air is passed through a solution of the composite catalyst to form the oxygen complex and the ethylene complex thereby to react them together.

7. A process according to any one of the preceding Claims, wherein the composite catalyst is dissolved in a solvent and the resulting solution of the catalyst is impregnated on a porous carrier, and oxygen or air and ethylene are passed therethrough to oxidise ethylene with the combined oxygen in the oxygen complex.

8. A process according to any one of the preceding Claims, wherein a basic (or electron-donating) compound selected from sulfolane, dimethylsulfolane, dimethylsulfoxide and dimethylformamide is added to a solution of said complex catalyst.

9. A process according to any one of Claims 1 to 7, wherein a basic (or electron-donating) compound selected from sulfolane, dimethylsulfolane, dimethylsulfoxide and dimethylformamide is used as a solvent for said composite catalyst.

**Patantensprüche**

1. Verfahren zur Herstellung von Azetaldehyd durch Oxydieren von Äthylen mit Sauerstoff in Gegenwart eines Metallkomplex-Katalysators, dadurch gekennzeichnet, daß der Metallkomplex-Katalysator ein zusammengesetzter Katalysator ist, der einen ersten Metallkomplex-Kataylsator nach der Formel $M_mX_n \cdot L_l$ enthalt, welcher geeignet ist, einen Sauerstoffkomplex durch koordinative Verbindung mit Sauerstoff zu bilden, wobei M ein Übergangsmetall ist, das mindestens einer der Gruppen I und IV bis VII und der Eisengruppe in Gruppe VIII der Tafel des Periodensystems angehört, und X ein Anion, der Ligand L eine organische Schwefelverbindung und jeder der Indices m, n, und l eine positive ganze Zahl von 1 bis 4 darstellt, sowie einen zweiten Metallkomplex-Katalysator nach der Formel $M'_{m'} X_{n'} \cdot L'_{l'}$, welcher geeignet ist, einen Äthylenkomplex durch koordinative Verbindung mit Äthylen zu bilden, wobei M' ein Übergangsmetall aus der Platingruppe in Gruppe VIII der Tafel des Periodensystems ist, X ein Anion, der Ligand L' mindestens eine Verbindung ausgewählt aus einem Nitril, einer organischen Fluorverbindung und Alkoxyl-, Alkyl- und Amidderivaten von Schwefelsäure und schwefeliger Säure, und jeder der Indices m', n' und l' eine positive ganze Zahl von 1 bis 4 darstellt, und daß das Verfahren Schritte enthält, in denen der erste Metallkomplex-Katalysator zur Bildung eines Sauerstoffkomplexes mit Sauerstoff in Kontakt gebracht wird, der zweite Metallkomplex-Katalysator zur Bildung eines Äthylenkomplexes mit Äthylen in Kontakt gebracht wird und der Sauerstoffkomplex mit dem Äthylenkomplex erbindung zur Erzeugung von Azetaldehyd zur Reaktion gebracht wird.

2. Verfahren nach Anspruch 1, dadurch gekennzeichnet, daß X ein Anion ist, das aus der Gruppe von $Cl^-$, $Br^-$, $I^-$, $BF_4^-$, $PF_6^-$, $SO_4^{2-}$ und $CH_3COO^-$ -Anionen gewählt wird.

3. Verfahren nach Anspruch 1 oder 2, dadurch gekennzeichnet, daß der Ligand L mindestens eine

Verbindung ist, die aus Alkoxyl-, Alkyl- und Amidderivaten von Schwefelsäure und schwefeliger Säure gewählt wird.

4. Verfahren nach einem der vorangehenden Ansprüche, dadurch gekennzeichnet, daß als Lösungsmittel für den ersten Metallkomplex und für den zweiten Metallkomplex mindestens eine Verbindung verwendet wird, die aus aliphatischen, aromatischen und alizyklischen Kohlenwasserstoffen, sauerstoffhaltigen organischen Verbindungen und stickstoffhaltigen organischen Verbindungen gewählt wird.

5. Verfahren nach einem der vorangehenden Ansprüche, dadurch gekennzeichnet, daß der Ligand L oder L' eine Flüssigkeit ist, und daß die als Ligand L oder L' verwendete Verbindung auch als Lösungsmittel für die Komplexe verwendet wird.

6. Verfahren nach einem der vorangehenden Ansprüche, dadurch gekennzeichnet, daß Sauerstoff oder Luft durch eine Lösung des zusammengesetzten Katalysators geleitet wird, um den Sauerstoffkomplex und den Äthylenkomplex zu bilden und diese dadurch miteinander zur Reaktion zu bringen.

7. Verfahren nach einem der vorangehenden Ansprüche, dadurch gekennzeichnet, daß der zusammengesetzte Katalysator in einem Lösungsmittel aufgelöst und mit der entstandenen Lösung des Katalysators ein poröser Träger inprägniert wird, und daß durch denselben Sauerstoff oder Luft und Äthylen geleitet wird, um das Äthylen mit dem im Sauerstoffkomplex verbundenen Sauerstoff zu oxydieren.

8. Verfahren nach einem der vorangehenden Ansprüche, dadurch gekennzeichnet, daß eine basische (oder Elektronen abgebende) Verbindung, die aus der Gruppe von Sulfolan, Dimethylsulfolan, Dimethylsulfoxid und Dimethylformamid gewählt ist, einer Lösung des komplexen Katalysators zugesetzt wird.

9. Verfahren nach einem der Ansprüche 1 bis 7, dadurch gekennzeichnet, daß eine basische (oder Elektronen abgebende) Verbindung, die aus der Gruppe von Sulfolan, Dimethylsulfolan, Dimethylsulfoxid und Dimethylformamid gewählt ist, als Lösungsmittel für den komplexen Katalysator verwendet wird.

**Revendications**

1. Un procédé pour la production d'acétaldéhyde par oxydation de l'éthylène avec de l'oxygène en présence d'un complexe métallique catalytique, caractérisé en ce que le complexe métallique catalytique est un catalyseur composite comprenant un premier complexe métallique catalytique de formule $M_mX_n{\cdot}L_l$ capable de former un complexe oxygéné par coordination avec l'oxygène,

dans laquelle M représente un métal de transition appartenant à au moins un groupe choisi parmi les groupes I et IV à VII et parmi le groupe du fer du groupe VIII de la classification periodique; X représente un anion; le ligand L représente un composé organique phosphoré; et chacun de m, n et l represente indépendamment un entier positif de 1 à 4, et un second complexe métallique catalytique de formule $M'_{m'}X_{n'}{\cdot}L'_{l'}$, capable de former un complexe éthylénique par coordination avec l'éthylène,

dans laquelle M' représente un métal de transition du groupe du platine du groupe VIII de la classification périodique; X représente un anion; le ligand L' représente au moins un composé choisi parmi un nitrile, un composé organique fluoré et les dérivés alcoxy, alkyles et amides de l'acide phosphoreux et de l'acide phosphorique; et chacun de m', n' et l' représente indépendendamment un entier positif de 1 à 4,

le procédé comprenant le contact du premier complexe métallique catalytique avec de l'oxygène pour former un complexe oxygéné, le contact du second complexe métallique catalytique avec de l'éthylène pour former un complexe éthylénique et la réaction conjointe dudit complexe oxygéné et dudit complexe éthylénique pour produire de l'acétaldéhyde.

2. Un procédé selon la revendication 1 dans lequel X est un anion choisi parmi les anions $Cl^-$, $Br^-$, $I^-$, $BF_4^-$, $PF_6^-$, $SO_4^{2-}$ et $CH_3COO^-$.

3. Un procédé selon la revendication 1 ou 2 dans lequel le ligand L est au moins un composé choisi parmi les dérivés alcoxy, alkyles et amides de l'acide phosphoreux et de l'acide phosphorique.

4. Un procédé selon l'une quelconque des revendications précédentes dans lequel, comme solvant dudit premier complexe métallique et dudit second complexe métallique, on utilise au moins un composé choisi les hydrocarbures aliphatiques, aromatiques et alicycliques, les composés organiques oxygénés, les composés organiques halogénés et les composés organiques azotés.

5. Un procédé selon l'une quelconque des revendications précédentes dans lequel le ligand L ou L' est un liquide et le composé utilisé comme ligand L ou L' est également utilisé comme solvant desdits complexes.

6. Un procédé selon l'une quelconque des revendications précédentes dans lequel on fait passer de l'oxygène ou de l'air à travers und solution du catalyseur composite pour former le complexe oxygéné et le complexe éthylénique afin de les faire réagir ensemble.

7. Un procédé selon l'une quelconque des revendications précédentes dans lequel le catalyseur composite est dissous dans un solvant et on imprège un support poreux de la solution obtenue du catalyseur et on fait passer à travers de l'oxygène ou de l'air et de l'éthylène pour oxyder l'éthylène avec l'oxygène combiné dans le complexe oxygéné.

8. Un procédé selon l'une quelconque des revendications précédentes dans lequel un composé basique (ou donneur l'électrons) choisi parmi le sulfolane, le diméthylsulfolane, le diméthylsulfoxyde et le diméthylformamide est ajouté à une solution dudit complexe catalytique.

9. Un procédé selon l'une quelconque des revendications 1 à 7 dans lequel un composé basique (ou donneur d'électrons) choisi parmi le sulfolane, le diméthylsulfolane, le diméthylsulfoxyde et le diméthylformamide est utilisé comme solvant dudit complexe catalytique.